# EUROPEAN PATENT SPECIFICATION

(11) **EP 0 962 814 B1**
(45) Date of publication and mention of the grant of the patent: **29.01.2003**
(21) Application number: 99201626.1
(22) Date of filing: 25.05.1999
(51) Int. Cl.: G03C 1/498, C07C 51/41

(54) **Recording material with improved shelf-life producing prints upon thermal development with improved archivability**
Aufzeichnungsmaterial mit verbesserter Lagerfähigkeit, das Ausdrucke mit verbesserter Archivierbarkeit nach thermischer Entwicklung ergibt
Produit d'enregistrement ayant une aptitude au stockage amélioré produisant des imprimées à l'archivabilité améliorée après développement thermique

(30) Priority: 06.06.1998 EP 98201962
(43) Date of publication of application: 08.12.1999
(73) Proprietor: AGFA-GEVAERT, 2640 Mortsel (BE)
(72) Inventor: Geuens, Ingrid, 2640 Mortsel (BE); Gilliams, Yvan, 2640 Mortsel (BE); Bollen, Dirk, 2640 Mortsel (BE); Hoogmartens, Yvan, 2640 Mortsel (BE); Bellens, André, 2640 Mortsel (BE)

(56) References cited:
- EP-A- 0 754 969
- EP-A- 0 848 286

## Description

### Field of the invention

The present invention relates to recording materials with improved shelf-life and prints produced therewith with improved archivability.

### Background of the invention.

Thermal imaging or thermography is a recording process wherein images are generated by the use of thermal energy. Such recording materials become photothermographic upon incorporating a photosensitive agent which after exposure to UV, visible or IR light is capable of catalyzing or participating in a thermographic process bringing about changes in colour or optical density.

US 4,273,723 discloses a process for preparing a silver salt of a fatty acid with 12 to 24 carbon atoms consisting essentially of reacting an alkali metal salt of the fatty acid with a water-soluble silver salt, and wherein the reaction is effected in a reaction system consisting essentially of (I) the alkali metal salt of the fatty acid, (II) the water-soluble silver salt, (III) at least one water-soluble or partially water-soluble C₃-C₈ alcohol and (IV) water, the volume ratio of the component (III) to the component (IV) being 1/5 to 5/1.

GB-A 1,378,734 discloses a process of producing a silver salt of an organic carboxylic acid conducted in the presence of a soluble mercury compound and/or a soluble lead compound.

EP-A 754 969 discloses a process for producing a suspension of particles containing a substantially light-insensitive silver salt of an organic carboxylic acid, comprising simultaneous metered addition of an aqueous solution or suspension of an organic carboxylic acid or its salt; and an aqueous solution of a silver salt to an aqueous liquid, wherein the metered addition of the aqueous solution or suspension of the organic carboxylic acid or its salt; and/or the aqueous solution of the silver salt is regulated by the concentration of silver ions or the concentration of anions of the silver salt in the aqueous liquid. Research Disclosure number 17029, published in June 1978, gives a survey of different methods of preparing organic heavy metal salts in section II.

The association of silver stearate with mercury or lead ions, particularly mercury ions, according to the teaching of GB 1,378,734, is environmentally undesirable and infringes governmental regulations.

Recording materials with prior art silver stearate exhibit poor shelf-life and prints produced therewith exhibit poor archivability, particularly as regards increase in Dₘₐₓ.

### Objects of the invention.

It is therefore an object of the invention to provide materials with an improved shelf-life.

It is a further object of the present invention to provide recording materials whose prints exhibit improved archivability.

It is a still further object of the present invention to provide production processes for substantially light-insensitive organic silver salt comprising silver stearate.

Further objects and advantages of the invention will become apparent from the description hereinafter.

### Summary of the invention

Surprisingly it has been found that recording materials comprising a support and a thermosensitive element comprising silver stearate with a higher crystallinity than prior art silver stearate, an organic reducing agent therefor in thermal working relationship therewith and a binder exhibit a marked improvement in shelf-life and that prints produced therewith exhibit a marked improvement in archivability over prior art recording materials with silver stearate.

The above mentioned objects are realized with a recording material comprising a support and a thermosensitive element containing silver stearate, an organic reducing agent therefor in thermal working relationship therewith and a binder, characterized in that the silver stearate is not associated with mercury and/or lead ions and when the recording material is irradiated with a copper Kα₁ X-ray source the ratio of the sum of the peak heights of the X-ray diffraction lines attributable to silver stearate at Bragg angles, 2Θ, of 3.62°, 5.45°, 7.30°, 9.04°, 10.97° and 12.71° to the sum of the peak heights of the X-ray diffraction lines at Bragg angles, 2Θ, of 25.60°, 35.16° and 43.40° of NIST standard 1976, rhombohedral Al₂O₃, determined with the same X-ray diffractometer in the same state of adjustment on a sample of the recording material and a sample of the NIST standard 1976 cut to fit a sample holder of the X-ray diffractometer, divided by the square root of the quantity of silver in the recording material, expressed in g per m², is greater than 2.2m/g^{0.5}.

A production process for a dispersion of particles of substantially light-insensitive organic silver salt containing silver stearate in a substantially solvent-free aqueous medium is further provided according to the present invention comprising the steps of: i) producing an aqueous dispersion of one or more organic acids containing stearic acid and an anionic surfactant; ii) substantially neutralizing the organic acids with aqueous alkali thereby forming organic acid salts containing a stearic acid salt; (iii) adding an aqueous solution of a silver salt to convert completely the organic acid salt(s) into their silver salts containing silver stearate, characterized in that the anionic surfactant is present in a molar ratio with respect to organic acid greater than 0.15 and the silver salt is added to produce organic silver salt(s) at a rate between 0.025mol/mol organic silver salt(s) • min and 2.25mol/mol organic silver salt(s) • min.

Particles of substantially light-insensitive organic silver salt containing silver stearate producible according to the above-mentioned process are also provided.

A recording process is further provided according to the present invention comprising the steps of: (i) bringing an outermost layer of the above-mentioned recording material in proximity with a heat source; and (ii) applying heat from the heat source imagewise to the recording material while maintaining proximity to the heat source to produce an image; and (iii) removing the recording material from the heat source.

Preferred embodiments of the invention are disclosed in the dependent claims.

### Detailed description of the invention.

In a preferred embodiment of the recording process, according to the present invention, the heat source is a thermal head with a thin film thermal head being particularly preferred.

### Substantially

By substantially light-insensitive is meant not intentionally light sensitive. By the terms substantially solvent-free aqueous medium and in the substantial absence or organic solvent is meant that solvent, if present, is miscible with water and present in amounts below 10% by volume of the aqueous medium.

### Thermosensitive element

The thermosensitive element, according to the present invention, comprises silver stearate, an organic reducing agent therefor in thermal working relationship therewith and a binder. The element may comprise a layer system in which the ingredients may be dispersed in different layers, with the proviso that the two ingredients are in reactive association with one another i.e. during the thermal development process the reducing agent must be present in such a way that it is able to diffuse to the silver stearate so that reduction of silver stearate to silver can occur.

In a preferred embodiment of the present invention the thermosensitive element further comprises a photosensitive species capable upon exposure of forming a species capable of catalyzing reduction of the silver stearate.

### Silver stearate characterization

The silver stearate in the recording material, of the present invention, is characterized in that when the recording material is irradiated with a copper Kα₁ X-ray source the ratio of the sum of the peak heights of the X-ray diffraction lines attributable to silver stearate at Bragg angles, 2Θ, of 3.62°, 5.45°, 7.30°, 9.04°, 10.97° and 12.71° to the sum of the peak heights of the X-ray diffraction lines at Bragg angles, 2Θ, of 25.60°, 35.16° and 43.40° of NIST (National Institute of Standards, Gaithersburg, MD 20899-0001, USA) standard 1976, rhombohedral Al₂O₃, determined with the same X-ray diffractometer in the same state of adjustment on a sample of the recording material and a sample of the NIST standard 1976 cut to fit a sample holder of the X-ray diffractometer, divided by the square root of the quantity of silver in the recording material, expressed in g per m², is greater than 2.2m/g^{0.5}, which is referred to in the detailed description of the present invention as the crystallinity of silver stearate. In a preferred embodiment of the present invention, the crystallinity of silver stearate is greater than 3.0m/g^{0.5}.

The crystallinity of the silver stearate in the recording material of the present invention is obtained by determining X-ray diffraction spectra on sheets of a particular recording material and of the NIST standard 1976 cut to fit the sample holder of the X-ray diffractometer used, subtracting the background using standard techniques, determining the peak heights (maxima) of the diffraction peaks, determining for the sample of recording material the sum of the peak heights (maxima), Kₘₐₜₑᵣᵢₐₗ, of the XRD lines attributable to silver stearate at Bragg angles, 2Θ, of 3.62°, 5.45°, 7.30°, 9.04°, 10.97° and 12.71°, determining for the sample of NIST standard 1976 the sum of the peak heights (maxima), K₁₉₇₆, of the X-ray diffraction lines at Bragg angles, 2Θ, of 25.60°, 35.16° and 43.40°, calculating the ratio of Kₘₐₜₑᵣᵢₐₗ/K₁₉₇₆ for the recording material, determining the concentration of silver C_{Ag} present in the recording material in grams per square metre of material and finally normalizing the ratio Kₘₐₜₑᵣᵢₐₗ/K₁₉₇₆ with √C_{Ag} to give Kₘₐₜₑᵣᵢₐₗ/(K₁₉₇₆ x √C_{ag}), which is a relative crystallinity for the silver stearate in the recording material concerned. The exact positions of the peaks attributable to silver stearate can vary within 0.3° of the angles given above. In such cases the peak height should be taken as the actual peak height of the peak and not the height of the peak at the angle given above.

The concentration of silver present in the recording material can be determined by any known technique e.g. non-destructive methods such as X-ray fluorescence and destructive methods such as dissolution of the silver salt followed by standard volumetric techniques for the determination of silver, such as described in R. Belcher and A.J. Nutten, Quantitative Inorganic Analysis, 2nd Edition, Butterworths, London (1960), pages 201-219.

### Organic silver salt particles containing silver stearate

Organic silver salt particles containing silver stearate may contain up to 100mol% of silver stearate. They preferably contain at least 50mol% of silver stearate. Preferred substantially light-insensitive organic silver salts used in the present invention are silver salts of organic carboxylic acids for use in the recording materials of the present invention together with silver stearate are silver salts of other aliphatic carboxylic acids known as fatty acids, wherein the aliphatic carbon chain has preferably at least 12 C-atoms, e.g. silver laurate, silver stearate, silver hydroxystearate, silver behenate and silver arichidate. Silver salts of modified aliphatic carboxylic acids with thioether group as described e.g. in GB-P 1,111,492 and other organic silver salts as described in GB-P 1,439,478, e.g. silver benzoate, may likewise be used to produce a thermally developable silver image. Combinations of different organic silver salts may also be used in the present invention.

The silver stearate of the present invention is not associated with mercury and/or lead ions. This means that mercury/and or silver ions are not intentionally added at any point during the preparation process and therefore are not intentionally associated with the silver stearate in the recording material of the present invention.

### Preparation of aqueous dispersions of organic silver salt particles containing silver stearate in the substantial absence of solvent

A production process for a dispersion of particles of substantially light-insensitive organic silver salt containing silver stearate in a substantially solvent-free aqueous medium is provided according to the present invention comprising the steps of: i) producing an aqueous dispersion of one or more organic acids containing stearic acid and an anionic surfactant; ii) substantially neutralizing the organic acids with aqueous alkali thereby forming organic acid salts containing a stearic acid salt; (iii) adding an aqueous solution of a silver salt to convert completely the organic acid salts into their silver salts containing silver stearate, characterized in that the anionic surfactant is present in a molar ratio with respect to organic acid greater than 0.15 and the silver salt is added to produce organic silver salt(s) at a rate between 0.025mol/mol organic silver salt(s) • min and 2.25mol/mol organic silver salt(s) • min. In preferred embodiments of the above production process the anionic surfactant is present in a molar ratio with respect to organic carboxylic acid greater than 0.25 and the silver salt is added at a rate between 0.03mol/mol organic silver salt(s) • min and 0.7mol/mol organic silver salt(s) • min, with a molar ratio of anionic surfactant with respect to organic acid greater than 0.3 and a rate of silver salt addition of between 0.04mol/mol organic silver salt(s) • min and 0.3mol/mol organic silver salt(s) • min being particularly preferred.

In a preferred embodiment, step (iii) of the production process of the present invention is carried out such that part of the solution of acid salts produced in step (ii) of the process is present in the reaction vessel prior to silver salt solution addition and part thereof is added simultaneously with the addition of the silver salt solution, with about 25 to 50% of the solution of acid salts produced in step (ii) being in the reaction vessel prior to silver salt addition being particularly preferred.

Preferred anionic surfactants for use in the above-mentioned process are alkali or ammonium salts of an acid selected from the group consisting of: alkylsulfonic acids, alkarylsulfonic acids, aralkylsulfonic acids, arylsulfonic acids, alkylsulfuric acids, aralkylsulfuric acids, arylsulfuric acids, alkarylsulfuric acids and organic carboxylic acids. Alkali or ammonium salts of alkylarylsulfonic acids are preferred with alkali or ammonium salts of alkylbenzene sulfonic acids being particularly preferred. Suitable anionic surfactants for use in the above-mentioned process are:
- Surfactant Nr. 1 =: MARLON™ A-396, a sodium alkyl-phenylsulfonate from Hüls;
- Surfactant Nr. 2 =: ERKANTOL™ BX, a sodium diisopropylnaphthalenesulfonate from BAYER;
- Surfactant Nr. 3 =: ULTRAVON™ W, a sodium arylsulfonate from Ciba-Geigy.

In the above-mentioned process the pH used is sufficiently low to avoid the oxidation of silver ions to silver oxide or silver hydroxide for which a pH below 10 is usually required, the process temperature is chosen such that it is above the melting point of the organic acid(s) used, about 70°C in the case of stearic acid and the process is carried out with stirring, the stirring rate being dependent upon: the size of the stirrer relative to the reaction vessel, the type of stirrer used, avoidance of silver oxide or silver hydroxide formation due to insufficient mixing and avoidance of foaming, it being usually between 200 and 1000rpm. Furthermore, a slight excess of an organic acid, for example 2 mol% of stearic acid, is preferred.

The size of the substantially light-insensitive organic silver salt particles containing silver stearate can be varied by varying the rate of silver salt addition, the concentration of anionic surfactant and the temperature, the equivalent diameter of the particles increasing with decreasing addition rate, decreasing anionic surfactant concentration and increasing temperature.

In a further preferred embodiment of the above-mentioned process the dispersion of particles of substantially light-insensitive organic silver salt containing silver stearate is subjected to ultrafiltration. The ultrafiltration process removes ionic species and concentrates the dispersion of substantially light-insensitive organic silver salt containing silver stearate by filtration through a cartridge-filter with a pore size sufficiently small to remove the salt produced upon the formation of the organic silver salt without removing the organic silver salt. Cartridge-filters with 10 000 to 500 000 MW have been found to be suitable for this purpose. In order to maintain the stability of the dispersion of substantially light-insensitive organic silver salt containing silver stearate during ultrafiltration it is necessary to maintain a minimum anionic surfactant concentration, but the counterion of the anionic surfactant can be changed, if the presence of the original counterion is undesirable in the recording material. For example the sodium ions in Surfactant nr 1 can be replaced by ammonium ions by washing with an ammonium nitrate solution during the ultrafiltration process and the sodium ion concentration reduced to below 100ppm.

The above-mentioned process produces substantially light-insensitive organic silver salt particles containing silver stearate in which the silver stearate has a crystallinity, as defined above, greater than 2.2m/g^{0.5}.

### Substantially light-insensitive organic silver salt dispersions

In the case of dried particles of organic silver salt comprising silver stearate with higher crystallinity, as defined above, it has been found that recording materials, according to the present invention, can be produced, if dispersions thereof are produced using dispersion techniques in which the particles themselves are subjected to as little damage as possible commensurate with achieving a satisfactory dispersion quality e.g. using microfluidizers, ultrasonic apparatuses, rotor stator mixers etc.

### Reducing agents

Suitable organic reducing agents for the reduction of organic silver salt particles containing silver stearate are organic compounds containing at least one active hydrogen atom linked to O, N or C, such as is the case with, aromatic di- and tri-hydroxy compounds. Catechol-type reducing agents, i.e. reducing agents containing at least one benzene nucleus with two hydroxy groups (-OH) in ortho-position, such as catechol, 3-(3,4-dihydroxyphenyl) propionic acid, 1,2-dihydroxybenzoic acid, gallic acid and esters e.g. methyl gallate, ethyl gallate, propyl gallate, tannic acid, and 3,4-dihydroxy-benzoic acid esters are preferred, with those described in EP-B 692 733 and EP-A 903 625 being particularly preferred.

Other suitable reducing agents, particularly for photothermographic recording materials, are sterically hindered phenols, bisphenols and sulfonamidophenols.

Combinations of reducing agents may also be used that on heating become reactive partners in the reduction of the substantially light-insensitive organic silver salt containing silver stearate. For example, combinations of sterically hindered phenols with sulfonyl hydrazide reducing agents such as disclosed in US-P 5,464,738; trityl hydrazides and formyl-phenyl-hydrazides such as disclosed in US-P 5,496,695; trityl hydrazides and formyl-phenyl-hydrazides with diverse auxiliary reducing agents such as disclosed in US-P 5,545,505, US-P 5.545.507 and US-P 5,558,983; acrylonitrile compounds as disclosed in US-P 5,545,515 and US-P 5,635,339; and 2-substituted malonodialdehyde compounds as disclosed in US-P 5,654,130.

### Film-forming binders of the thermosensitive element

The film-forming binder of the thermosensitive element containing organic silver salt particles containing silver stearate may be all kinds of natural, modified natural or synthetic resins or mixtures of such resins, in which the organic silver salt particles containing silver stearate can be dispersed homogeneously either in aqueous or solvent media: e.g. cellulose derivatives such as ethylcellulose, cellulose esters, e.g. cellulose nitrate, carboxymethylcellulose, starch ethers, galactomannan, polymers derived from α,β-ethylenically unsaturated compounds such as polyvinyl chloride, after-chlorinated polyvinyl chloride, copolymers of vinyl chloride and vinylidene chloride, copolymers of vinyl chloride and vinyl acetate, polyvinyl acetate and partially hydrolyzed polyvinyl acetate, polyvinyl alcohol, polyvinyl acetals that are made from polyvinyl alcohol as starting material in which only a part of the repeating vinyl alcohol units may have reacted with an aldehyde, preferably polyvinyl butyral, copolymers of acrylonitrile and acrylamide, polyacrylic acid esters, polymethacrylic acid esters, polystyrene and polyethylene or mixtures thereof.

The above mentioned binders or mixtures thereof may be used in conjunction with waxes or "heat solvents" also called "thermal solvents" or "thermosolvents" improving the reaction speed of the redox-reaction at elevated temperature.

### Toning agent

In order to obtain a neutral black image tone in the higher densities and neutral grey in the lower densities the thermosensitive element contains preferably in admixture with the organic silver salt particles containing silver stearate and reducing agents a so-called toning agent known from thermography or photothermography.

Suitable toning agents are the phthalimides and phthalazinones within the scope of the general formulae described in US-P 4,082,901. Further reference is made to the toning agents described in US-P 3,074,809, 3,446,648 and 3,844,797. Other particularly useful toning agents are the heterocyclic toner compounds of the benzoxazine dione or naphthoxazine dione type as disclosed in GB-P 1,439,478, US-P 3,951,660 and US-P 5,599,647.

### stabilisers and antifoggants

In order to obtain improved shelf-life and reduced fogging, stabilizers and antifoggants may be incorporated into the recording materials of the present invention.

### Other additives

The recording material may contain in addition to the ingredients mentioned above other additives such as free fatty acids, additional surfactants, antistatic agents, e.g. non-ionic antistatic agents including a fluorocarbon group as e.g. in F₃C(CF₂)₆CONH(CH₂CH₂O)-H, silicone oil, e.g. BAYSILONE™ Öl A (from BAYER AG, GERMANY), ultraviolet light absorbing compounds, white light reflecting and/or ultraviolet radiation reflecting pigments and/or optical brightening agents.

### Support

The support for the thermosensitive element according to the present invention may be transparent, translucent or opaque, e.g. having a white light reflecting aspect and is preferably a thin flexible carrier e.g.polypropylene, polycarbonate or polyester, e.g. polyethylene terephthalate.

The support may be in sheet, ribbon or web form and subbed if need be to improve the adherence to the thereon coated thermosensitive element. The support may be made of an opacified resin composition. Should a transparent base be used, the base may be colourless or coloured, e.g. having a blue colour. One or more backing layers may be provided to control physical properties such as curl and static.

### Outermost layer

The outermost layer of the recording material may in different embodiments of the present invention be the outermost layer of the thermosensitive element, a protective layer applied to the thermosensitive element or a layer on the opposite side of the support to the thermosensitive element.

### Protective layer

According to a preferred embodiment of the recording material, according to the present invention, the thermosensitive element is provided with a protective layer to avoid local deformation of the thermosensitive element and to improve resistance against abrasion.

The protective layer preferably comprises a binder, which may be solvent-soluble, solvent-dispersible, water-soluble or water-dispersible. Among the solvent-soluble binders polycarbonates as described in EP-A 614 769 are particularly preferred. However, water-soluble or water-dispersible binders are preferred for the protective layer, as coating can be performed from an aqueous composition and mixing of the protective layer with the immediate underlayer can be avoided by using a solvent-soluble or solvent-dispersible binder in the immediate underlayer.

A protective layer according to the present invention may comprise in addition a thermomeltable particle optionally with a lubricant present on top of the protective layer as described in WO 94/11199. In a preferred embodiment at least one solid lubricant having a melting point below 150°C and at least one liquid lubricant in a binder is present, wherein at least one of the lubricants is a phosphoric acid derivative.

### Crosslinking agents for outermost layer

The outermost layer according to the present invention may be crosslinked. Crosslinking can be achieved by using crosslinking agents such as described in WO 95/12495 for protective layers, e.g. tetra-alkoxysilanes, polyisocyanates, zirconates, titanates, melamine resins etc., with tetraalkoxysilanes such as tetramethylorthosilicate and tetraethylorthosilicate being preferred.

### Matting agents for outermost layer

The outermost layer of the recording material according to the present invention may comprise a matting agent. Suitable matting agents are described in WO 94/11198 and include e.g. talc particles and optionally protrude from the outermost layer.

### Lubricants for outermost layer

Solid or liquid lubricants or combinations thereof are suitable for improving the slip characteristics of the recording materials according to the present invention.

Solid lubricants which can be used according to the present invention are polyolefin waxes, ester waxes, polyolefin-polyether block copolymers, amide waxes, polyglycols, fatty acids, fatty alcohols, natural waxes and solid phosphoric acid derivatives. Preferred solid lubricants are thermomeltable particles such as those described in WO 94/11199.

Liquid lubricants which can be used according to the present invention according to the present invention are fatty acid esters such as glycerine trioleate, sorbitan monooleate and sorbitan trioleate, silicone oil derivatives and phosphoric acid derivatives.

### Photosensitive species

A preferred photosensitive species capable upon exposure of forming species capable of catalyzing reduction of the silver stearate of the present invention is silver halide.

The photosensitive silver halide used in the present invention may be employed in a range of 0.1 to 100 mol percent; preferably, from 0.2 to 80 mol percent; particularly preferably from 0.3 to 50 mol percent; especially preferably from 0.5 to 35 mol %; and especially from 1 to 12 mol % of substantially light-insensitive organic silver salt.

The silver halide may be any photosensitive silver halide such as silver bromide, silver iodide, silver chloride, silver bromoiodide, silver chlorobromoiodide, silver chlorobromide etc. The silver halide may be in any form which is photosensitive including, but not limited to, cubic, orthorhombic, tabular, tetrahedral, octagonal etc. and may have epitaxial growth of crystals thereon.

The silver halide used in the present invention may be employed without modification. However, it may be chemically sensitized with a chemical sensitizing agent such as a compound containing sulphur, selenium, tellurium etc., or a compound containing gold, platinum, palladium, iron, ruthenium, rhodium or iridium etc., a reducing agent such as a tin halide etc., or a combination thereof. The details of these procedures are described in T.H. James, "The Theory of the Photographic Process", Fourth Edition, Macmillan Publishing Co. Inc., New York (1977), Chapter 5, pages 149 to 169.

### Spectral sensitizers

The thermosensitive element, according to the present invention, may contain an infra-red sensitizer, an ultra-violet light sensitizer or a visible light sensitizer. Suitable sensitizers include cyanine, merocyanine, styryl, hemicyanine, oxonol, hemioxonol and xanthene dyes. According to the present invention the thermosensitive element may further include a supersensitizer.

### Antihalation dyes

In addition to the ingredients, the recording materials used in the present invention may also contain antihalation or acutance dyes which absorb light which has passed through the photosensitive thermally developable photographic material, thereby preventing its reflection. Such dyes may be incorporated into the thermosensitive element or in any other layer of the recording material of the present invention.

### Coating

The coating of any layer of the recording material of the present invention may proceed by any coating technique e.g. such as described in Modern Coating and Drying Technology, edited by Edward D. Cohen and Edgar B. Gutoff, (1992) VCH Publishers Inc. 220 East 23rd Street, Suite 909 New York, NY 10010, U.S.A.

### Thermographic processing

Thermographic imaging is carried out by the image-wise application of heat either in analogue fashion by direct exposure through an image of by reflection from an image, or in digital fashion pixel by pixel either by using an infra-red heat source, for example with a Nd-YAG laser or other infra-red laser, with a thermographic material preferably containing an infra-red absorbing compound, or by direct thermal imaging with a thermal head.

In thermal printing image signals are converted into electric pulses and then through a driver circuit selectively transferred to a thermal printhead. The thermal printhead consists of microscopic heat resistor elements, which convert the electrical energy into heat via Joule effect. Such thermal printing heads may be used in contact or close proximity with the recording material. The operating temperature of common thermal printheads is in the range of 300 to 400°C and the heating time per picture element (pixel) may be less than 1.0ms, the pressure contact of the thermal printhead with the recording material being e.g. 200-500g/cm² to ensure a good transfer of heat.

In order to avoid direct contact of the thermal printing heads with the outermost layer on the same side of the support as the thermosensitive element when this outermost layer is not a protective layer, the image-wise heating of the recording material with the thermal printing heads may proceed through a contacting but removable resin sheet or web wherefrom during the heating no transfer of recording material can take place.

Activation of the heating elements can be power-modulated or pulse-length modulated at constant power. The image-wise heating can be carried out such that heating elements not required to produce an image pixel generate an amount of heat (Hₑ) in accordance with the following formula: 0.5 H_{D} < Hₑ < H_{D} wherein H_{D} represents the minimum amount of heat required to cause visible image formation in the recording material.

EP-A 654 355 discloses a method for making an image by image-wise heating by means of a thermal head having energizable heating elements, wherein the activation of the heating elements is executed duty cycled pulsewise. EP-A 622 217 discloses a method for making an image using a direct thermal imaging element producing improvements in continuous tone reproduction.

Image-wise heating of the recording material can also be carried out using an electrically resistive ribbon incorporated into the material. Image- or pattern-wise heating of the recording material may also proceed by means of pixel-wise modulated ultra-sound.

### Photothermographic processing

Photothermographic recording materials, according to the present invention, may be exposed with radiation of wavelength between an X-ray wavelength and a 5 microns wavelength with the image either being obtained by pixel-wise exposure with a finely focused light source, a UV, visible or IR wavelength laser or a light emitting diode or by direct exposure to the object itself or an image therefrom with appropriate illumination.

For the thermal development of image-wise exposed photothermographic recording materials, according to the present invention, any sort of heat source can be used that enables the recording materials to be uniformly heated to the development temperature in a time acceptable for the application concerned.

### Industrial application

Thermographic and photothermographic imaging can be used for the production of transparencies and reflection type prints. Application of the present invention is envisaged in the fields of both graphics images requiring high contrast images with a very steep dependence of print density upon applied dot energy and continuous tone images requiring a weaker dependence of print density upon applied dot energy, such as required in the medical diagnostic field. In the hard copy field recording materials on a white opaque base are used, whereas in the medical diagnostic field black-imaged transparencies are widely used in inspection techniques operating with a light box.

The invention is illustrated hereinafter by way of invention examples and comparative examples. The percentages and ratios given in these examples are by weight unless otherwise indicated. The ingredients used in the invention and comparative examples, other than those mentioned above, are:
- as organic silver salt:
   - AgSt =: silver stearate;
- as binders:
   - K7598 =: type K7598, a calcium-free gelatine from AGFA-GEVAERT GELATINEFABRIEK vorm. KOEPFF & SÖHNE;
   - K17881 =: type K17881, a calcium-free gelatine from AGFA-GEVAERT GELATINEFABRIEK vorm. KOEPFF & SÖHNE;
   - K16353 =: type K16353, a calcium-free high viscosity gelatine from AGFA-GEVAERT GELATINEFABRIEK vorm. KOEPFF & SÖHNE;
   - LATEX 01 =: a 24% by weight aqueous latex of a polymer produced by copolymerizing a monomer mixture consisting of 42% by weight of n-butyl acrylate, 53% by weight of styrene, 2% by weight of itaconic acid and 3% by weight of CH₂=C(CH₃)CONH-(CH₂)₁₀-CONHC₆H₄-p-SO₃K followed by desalting and adjusting to pH 5.4 with ammonia;
- as reducing agent:
   - R01 =: ethyl 3,4-dihydroxybenzoate;
- as toning agent:
   - T01: = 7-(ethylcarbonato)-benzo[e][1,3]oxazine-2,4-dione (see formula I below)

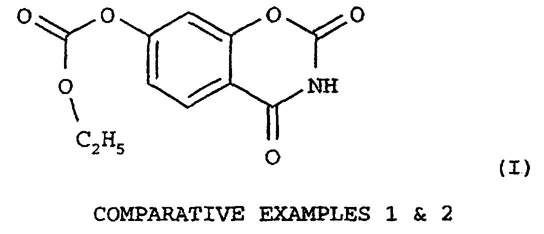

### preparation of prior art silver stearate according to RD 17029

In the preparation of Types I & II silver stearate, solution A was first prepared by adding 0.15 moles of solid sodium hydroxide to a dispersion of 0.1575 moles and 0.176 moles of stearic acid in 1L of deionized water at 75°C thereby producing a solution of sodium stearate with a pH of ca. 9. Solution B, 250mL of 0.6M aqueous silver nitrate acidified with 0.4g of 65% nitric acid at a temperature of 65°C, was then added with vigorous stirring to solution A in 15s while maintaining a temperature of 75°C. After 1 minute the resulting suspension of silver stearate was cooled to room temperature and had a pH of ca. 5 and a UAg of ca. 310mV. The silver stearate was filtered off under reduced pressure, washed twice each time with about 5L of deionized water and dried in a forced air drying cupboard at 40°C.

### preparation of silver stearate dispersions

The quantities of type I and type II silver stearates given in table 1 were dispersed with the quantities of deionized water and 10% solution of Surfactant Nr 1 given in table 1 first with an ULTRATURRAX™ mixer to obtain a predispersion and then through a MICROFLUIDICS™ M-110Y high pressure microfluidizer at a jet pressure of 350 bar to produce the final dispersions with concentrations of 11.5% and 11.3% respectively.

**Table 1**

| Comparative example nr | silver stearate | | quantity of deionized water [g] | quantity of 10% solution of Surfactant Nr 1 [g] |
|---|---|---|---|---|
| | type | quantity [g] | | |
| 1 | I | 58 | 342 | 100 |
| 2 | II | 60 | 340 | 100 |

### preparation of a tone modifier dispersion

The tone modifier dispersion was prepared by first dissolving 11g of K7598 in 69g of deionized water by first adding the gelatine, then allowing the gelatine to swell for 30 minutes and finally heating to 50°C. 20 g of T01 was added with ULTRATURRAX™ stirring to this gelatin solution at 50°C, and the stirring continued for a further 5 minutes. Finally the resulting dispersion was pumped through a DYNOMILL™ for 2 hours to produce the final tone modifier dispersion containing: 20% of T01 and 11% of gelatin.

### thermosensitive element

The thermosensitive emulsion was produced as follows: 2.341g of K7598 was allowed to swell for 30 minutes with deionized water (for the quantity used in the preparation of the thermographic emulsion for the particular recording material see table 2) and the resulting gel heated to 36°C. The following ingredients were then added with stirring: 5.699g of the tone modifier dispersion at 36°C, then 8.120g of LATEX 01 followed by 5 minutes stirring, the corresponding silver stearate dispersion (for quantity and silver stearate concentration therein used for the thermosensitive emulsion for the particular recording material see table 2) followed by 5 minutes stirring, 12.35g of a 10.95% ethanol solution of R01 at 45°C and finally 2.880g of a 3.7% aqueous solution of formaldehyde.

The thermosensitive dispersions were then doctor blade-coated onto a 175µm subbed PET support and dried for 10 minutes at 50°C thereby producing the thermosensitive elements of COMPARATIVE EXAMPLES 1 & 2.

**Table 2**

| Comparative example nr | quantity of water [g] | silver stearate dispersion | | |
|---|---|---|---|---|
| | | AgSt type | concentration (%) | quantity [g] |
| 1 | 20.140 | I | 11.504 | 37.470 |
| 2 | 20.450 | II | 11.298 | 38.160 |

### determination of silver stearate crystallinity in the recording materials

The crystallinity of the silver stearate in the recording materials of COMPARATIVE EXAMPLES 1 & 2 was determined as follows:
i) 30mm diameter samples of the recording materials of COMPARATIVE EXAMPLES 1 & 2 and of NIST standard 1976 were cut from larger sheets using a punch;
ii) X-ray diffraction scans were then carried out using a SIEMENS D5000 X-ray diffractometer equipped with a copper Kα₁ X-ray source operating at 40keV and a current of 30mA with the samples in the sample holder thereof to scan the samples of COMPARATIVE EXAMPLES 1 & 2 and NIST standard 1976, with the same X-ray diffractometer in exactly the same state of adjustment, in steps of 0.05 degrees at a rate of 1 step/s between Bragg angles, 2Θ, of 2° and 50° and the data processed using SIEMENS DIFFRAC™ AT software to produce X-ray diffraction spectra corrected for background and exact peak heights (maxima) of each X-ray diffraction peak;
iii) the Kₘₐₜₑᵣᵢₐₗ values were then determined for the recording materials of COMPARATIVE EXAMPLES 1 & 2 by adding up the peak heights (maxima) of the X-ray diffraction lines attributable to silver stearate at Bragg angles, 2Θ, of 3.62°, 5.45°, 7.30°, 9.04°, 10.97° and 12.71°;
iv) the K₁₉₇₆ value was determined for NIST standard 1976 by adding up the peak heights (maxima) of the X-ray diffraction lines at Bragg angles, 2Θ, of 25.60°, 35.16° and 43.40°;
v) the weights of silver in g/m², C_{Ag}, of the recording materials of COMPARATIVE EXAMPLES 1 & 2 were determined using a PHILIPS PW2400 wavelength dispersive X-ray fluorescence apparatus with a chromium K_{α} X-ray source operating at 60keV and a current of 50mA, which had been calibrated for silver using silver-containing samples for which the silver concentrations had been determined using standard volumetric titration techniques; and
vi) the crystallinity values for the silver stearate present in the recording materials of COMPARATIVE EXAMPLES 1 & 2 were determined using the expression: Kₘₐₜₑᵣᵢₐₗ/(K₁₉₇₆ x √C_{Ag}).
The crystallinity values for the silver stearate in the recording materials of COMPARATIVE EXAMPLES 1 & 2 are given in table 3.

### thermographic printing

The printer was equipped with a thin film thermal head with a resolution of 300 dpi and was operated with a line time of 19ms (the line time being the time needed for printing one line). During this line time the printhead received constant power. The average printing power, being the total amount of electrical input energy during one line time divided by the line time and by the surface area of the heat-generating resistors, was 1.6 mJ/dot and was sufficient to obtain maximum optical density in the thermographic recording materials of COMPARATIVE EXAMPLES 1 & 2.

During printing of the recording materials of COMPARATIVE EXAMPLES 1 & 2 the printhead was separated from the imaging layer by a thin intermediate material contacted with a slipping layer of a separable 5µm thick polyethylene terephthalate ribbon coated successively with a subbing layer, heat-resistant layer and the slipping layer (anti-friction layer) giving a ribbon with a total thickness of 6µm.

The maximum densities, Dₘₐₓ, and minimum densities, Dₘᵢₙ, of the prints given in table 3 were measured through a visible filter with a MACBETH™ TR924 densitometer in the grey scale steps corresponding to data levels of 64 and 0 respectively and are given in table 3 for COMPARATIVE EXAMPLES 1 & 2.

### shelf-life test

The shelf-life of the recording materials of COMPARATIVE EXAMPLES 1 & 2 was evaluated on the basis of the observed changes in minimum and maximum density measured through a visible filter using a MACBETH™ TR924 densitometer upon thermographic printing after heating the recording materials at 57°C in a relative humidity of 34% for 3 days in the dark. The results are given in table 3.

### archivability test

The achivability of prints made with the recording materials of COMPARATIVE EXAMPLES 1 & 2 was evaluated on the basis of the changes in minimum and maximum density, Dₘᵢₙ and Dₘₐₓ, measured through a visible filter using a MACBETH™ TR924 densitometer upon heating the prints at 57°C in a relative humidity (RH) of 34% for 3 days in the dark. The results are given in table 3.

**Table 3**

| Comparative example number | Silver stearate | | | fresh print characteristics | | archivability ΔDₘₐₓ/ΔDₘᵢₙ (vis) after 3d at 57°C/34% RV | shelf-life ΔDₘₐₓ/ΔDₘᵢₙ (vis) after 3d at 57°C/34% RV |
|---|---|---|---|---|---|---|---|
| | type | coating weight [g/m²] | crystallinity | Dₘₐₓ (vis) | Dₘᵢₙ (vis) | | |
| 1 | I | 3.99 | 2.01 | 3.20 | 0.08 | +0.43/0.00 | +0.51/0.00 |
| 2 | II | 3.66 | 1.44 | 3.21 | 0.07 | +0.46/+0.01 | +0.55/+0.02 |

### COMPARATIVE EXAMPLE 3

### preparation of prior art silver stearate according to EP-A 754 969

A sodium stearate solution was prepared by dissolving with stirring 27.0 g of sodium stearate in a mixture of 80 mL of 2-propanol and 285.5 mL of deionized water at 75°C to give a 7.19% by weight solution.

The silver stearate synthesis was carried out at a constant UAg of 400mV as follows: to a stirred solution of 30g of K17881 in 1000mL of distilled water at 71°C in a double walled reactor, several drops of a 2.94M aqueous solution of silver nitrate were added to adjust the UAg at the start of the reaction to 400mV and then 340g of the above-mentioned sodium stearate solution at a temperature of 75°C was metered into the reactor at a rate of 48mL/min and simultaneously a 3.792% by weight aqueous solution of silver nitrate was metered into the reactor, its addition rate being controlled by the quantity of the silver nitrate solution necessary to maintain a UAg of 400±5mV in the dispersing medium in the reactor. Both the sodium stearate and silver nitrate solutions were added to the dispersing medium via small diameter tubes positioned just under the surface of the dispersing medium. By the end of the addition step 0.080moles of sodium behenate and 0.087 moles of silver nitrate had been added. The mixture was then stirred for a further 30 minutes. The resulting silver stearate dispersion contained 1.79% by weight of silver behenate and 1.72% by weight of K17881.

5g of K16353 was added per 100g of silver stearate dispersion together with 6% of Surfactant Nr. 3 and the resulting dispersion doctor blade coated to a silver stearate coverage of 0.98g/m² after drying. The crystallinity of the silver stearate in the resulting material was determined as described for COMPARATIVE EXAMPLES 1 & 2 to be 1.80, see table 4.

**Table 4**

| Comparative example number | Silver stearate | | |
|---|---|---|---|
| | type | coating weight [g/m²] | crystallinity |
| 3 | VI | 0.98 | 1.80 |

Therefore the silver salt production process of EP-A 754 969 produces silver stearate with a crystallinity, as determined according to the present invention, below 2.2 m/g^{0.5} and hence outside the disclosure of the present invention.

### INVENTION EXAMPLES 1 to 3

### preparation of silver stearate dispersions in an aqueous medium in the absence of organic solvent using a single jet process

Aqueous dispersions of the silver stearate types III to V were produced as follows:
i) dispersing stearic acid (for quantity see table 5) with stirring at a given temperature (see table 5) in a mixture of deionized water (for quantity see table 5) and a 10% solution of Surfactant Nr 1 (for quantity see table 5) to produce a dispersion with a pH of about 4.2;
ii) then adding a quantity of 2M aqueous sodium hydroxide (for quantity see table 5) at the same temperature as the stearic acid dispersion with stirring over a particular time (see table 5 for the time of addition) thereby producing a clear solution with a pH of about 9.2 substantially containing sodium stearate;
iii) then metered addition of a particular quantity of an aqueous 1M silver nitrate solution (same quantity in moles as for sodium hydroxide) at the same temperature as the stearic acid dispersion with stirring at a particular rate (for rate given as moles/moles silver stearate • min see table 5) to convert the sodium stearate completely into silver stearate as a dispersion with a pH and UAg as given in table 5; and
iv) ultrafiltration with a 500000 MW polysulfone cartridge filter at room temperature to concentrate the resulting silver stearate dispersion (final AgSt-concentration and residual conductivity in mS/cm are given in table 6).
The volume average particle size as determined by a Coulter LS230 diffractometer is also given in table 6.

**Table 5**

| Invention example number | AgSt type | quantity of stearic acid [moles] | quantity of deionized water [L] | quantity of 10% sol. of Surfactant Nr 1 [L] | temperature [°C] | quantity of NaOH & AgNO₃ [moles] | addition time of 2M NaOH [min] | mol AgNO₃/mol AgSt • min | pH | UAg [mV] |
|---|---|---|---|---|---|---|---|---|---|---|
| 1 | III | 1.507 | 2.495 | 2.027 | 80 | 1.477 | 9.75 | 0.0650 | 6.61 | +314 |
| 2 | IV | 0.4 | 0.662 | 0.538 | 70 | 0.392 | 10 | 0.25 | 5.84 | +464 |
| 3 | V | 0.4 | 0.893 | 0.307 | 70 | 0.392 | 10 | 0.25 | 7.05 | +310 |

**Table 6**

| Invention example nr | AgSt type | ultrafiltration | | average particle size [nm] |
|---|---|---|---|---|
| | | residual conductivity [mS/cm] | % AgSt dispersion | |
| 1 | III | 3.2 | 16.86 | 339 |
| 2 | IV | 3.4 | 18.56 | |
| 3 | V | 2.0 | 19.31 | 3381 |

These dispersions of silver stearate were directly used in the preparation of the recording materials of INVENTION EXAMPLES 1 to 3.

### thermosensitive element

The thermosensitive elements of the recording materials of INVENTION EXAMPLES 1 to 3 were produced as described for the thermosensitive elements of the recording materials of COMPARATIVE EXAMPLES 1 & 2 except that the quantity of deionized water, the silver stearate type, concentration and quantity of dispersion used were as given in table 7 below.

**Table 7**

| Invention example nr | quantity of water [g] | silver stearate dispersion | | |
|---|---|---|---|---|
| | | AgSt type | concentration (%) | quantity [g] |
| 1 | 13.078 | III | 16.86 | 25.532 |
| 2 | 15.380 | IV | 18.56 | 23.230 |
| 3 | 16.290 | V | 19.31 | 22.320 |

The crystallinity values for the silver stearate in the recording materials of INVENTION EXAMPLES 1 to 3 determined as described for COMPARATIVE EXAMPLE 1 & 2 are given in table 8.

### thermographic evaluation

Thermographic printing with the recording materials of INVENTION EXAMPLES 1 to 3 and the evaluation thereof were carried out as described for the recording material of COMPARATIVE EXAMPLES 1 & 2. The results are summarized in table 8.

**Table 8**

| Invention example number | Silver stearate | | | fresh print characteristics | | archivability ΔDₘₐₓ/Dₘᵢₙ (vis) after 3d at 57°C/34% RV | shelf-life ΔDₘₐₓ/Dₘᵢₙ (vis) after 3d at 57°C/34% RV |
|---|---|---|---|---|---|---|---|
| | type | coating weight [g/m²] | crystallinity | Dₘₐₓ (vis) | Dₘᵢₙ (vis) | | |
| 1 | III | 3.37 | 2.48 | 3.24 | 0.07 | +0.25/0.00 | +0.19/0.00 |
| 2 | IV | 3.12 | 3.51 | 3.12 | 0.07 | +0.17/+0.01 | -0.19/0.00 |
| 3 | V | 3.81 | 3.16 | 3.38 | 0.07 | +0.24/+0.01 | -0.14/+0.01 |

These results show a considerable improvement in the shelf-life of recording materials of INVENTION EXAMPLES 1 to 3 and in the archivability of prints made therewith compared with the prior art recording materials of COMPARATIVE EXAMPLES 1 & 2 as demonstrated by a reduced increase in Dₘₐₓ while maintaining Dₘᵢₙ-stability. The recording materials of INVENTION EXAMPLES 1 to 3 only differ from those of COMPARATIVE EXAMPLES 1 & 2 in that they contain silver stearate with an increased crystallinity. This demonstrates the beneficial effect of increased silver stearate crystallinity on the stability of recording materials.

### COMPARATIVE EXAMPLE 4 & INVENTION EXAMPLE 4

The photothermographic recording materials of COMPARATIVE EXAMPLE 4 & INVENTION EXAMPLE 4 were prepared using the aqueous dispersions of type II and type IV silver stearate respectively prepared as described for COMPARATIVE EXAMPLE 2 and INVENTION EXAMPLE 2 respectively.

### photo-addressable thermally developable elements

The photo-addressable thermally developable element was produced as follows: 2.1g of K7598 was allowed to swell for 30 minutes in 8.4g of deionized water and the resulting gel heated to 36°C, then with stirring the following ingredients were added: 2.5g of an aqueous gelatinous emulsion of AgBr_{0.97}I_{0.03} (6% by weight in gelatin and 13.36% by weight in AgBr_{0.97}I_{0.03}), silver stearate dispersion ( for the quantity and concentration, see table 9), adjustment of the dispersion pH to 5.20 by addition of 1.25g of nitric acid, 2.240g of a 5.26% methanol solution of trimethylphenylammonium bromide perbromide, 9.804g of a 21.4% aqueous dispersion of LATEX01, 1.606g of an aqueous solution containing 18.68% of tribromomethylphenylsulfone, 9.3% of K7598 and 0.93% of Surfactant Nr 1 and finally 12.22g of a dispersion of 17.82% LOWINOX™ , 1.98% of Surfactant Nr 2 and 4.95% of phthalazine. The resulting dispersions were then doctor blade-coated onto a 175µm subbed PET support and dried for 10 minutes at 50°C to produce the photo-addressable thermally developable elements of the photothermographic recording materials of COMPARATIVE EXAMPLE 4 & INVENTION EXAMPLE 4 with a silver stearate coating weight of about 5.7g/m². These photo-addressable thermally developable elements were then overcoated using a 50µm doctor blade with 45g of an aqueous solution consisting of 6.67% of K7598, 0.44% of 4-methylphthalic acid and 4.44% of the ammonium salt of perfluoro-octanoic acid to produce, after drying at 50°C for 10 minutes, the photothermographic recording materials of COMPARATIVE EXAMPLE 4 & INVENTION EXAMPLE 4.

**Table 9**

| | silver stearate dispersion | | |
|---|---|---|---|
| | AgSt type | concentration [%] | quantity [g] |
| COMPARATIVE EXAMPLE 4 | II | 11.298 | 50.612 |
| Invention Example 4 | IV | 18.531 | 30.808 |

The crystallinity values for the silver stearate present in the photothermographic recording materials of COMPARATIVE EXAMPLE 4 & INVENTION EXAMPLE 4 determined as described for COMPARATIVE EXAMPLES 1 & 2 are given in table 10.

### photothermographic evaluation

The photothermographic recording materials of COMPARATIVE EXAMPLE 4 & INVENTION EXAMPLE 4 were then exposed to ultra-violet light through a test original in contact with the material in an AGFA-GEVAERT™ DL 1000 exposure apparatus followed by heating on a heated metal block for the times and at the temperatures given in Table 10 to produce a good image. The maximum densities, Dₘₐₓ, and minimum densities, Dₘᵢₙ, of fresh prints measured through a UV-filter and through a visible filter with a MACBETH™ TD905 densitometer in the grey scale steps corresponding to data levels of 64 and 0 respectively and are given for the photothermographic recording materials COMPARATIVE EXAMPLE 4 & INVENTION EXAMPLE 4 in tables 10 and 11 respectively.

### shelf-life test

The shelf-life of the recording materials of COMPARATIVE EXAMPLE 4 & INVENTION EXAMPLE 4 was evaluated on the basis of the changes in minimum and maximum density measured through a UV-filter and through a visible filter using a MACBETH™ TD905 densitometer upon thermographic printing after heating the recording materials at 35°C in a relative humidity of 80% for 3 days in the dark. The results are given in tables 10 and 11 respectively.

### Light box test

The stability of unprocessed sheets of the photothermographic recording materials of COMPARATIVE EXAMPLE 4 & INVENTION EXAMPLE 4 to room lighting was evaluated on the basis of the change in density measured through a UV filter using a MACBETH™ TD905 densitometer upon exposure on top of the white PVC window of a specially constructed light-box placed for 3 days in a Votsch conditioning cupboard set at 30°C and a relative humidity (RH) of 85%. Only a central area of the window 550mm long by 500mm wide was used for mounting the test materials to ensure uniform exposure.

The stainless steel light-box used was 650mm long, 600mm wide and 120mm high with an opening 610mm long and 560mm wide with a rim 10mm wide and 5mm deep round the opening, thereby forming a platform for a 5mm thick plate of white PVC 630mm long and 580mm wide, making the white PVC-plate flush with the top of the light-box and preventing light loss from the light-box other than through the white PVC-plate. This light-box was fitted with 9 Planilux™ TLD 36W/54 fluorescent lamps 27mm in diameter mounted length-wise equidistantly from the two sides, with the lamps positioned equidistantly to one another and the sides over the whole width of the light-box and with the tops of the fluorescent tubes 30mm below the bottom of the white PVC plate and 35mm below the materials being tested. The results are summarized in tables 10 and 11.

The results in tables 10 and 11 show a considerable improvement in the shelf-life stability of the photothermographic recording material of INVENTION EXAMPLES 4 compared with the photothermographic recording material of COMPARATIVE EXAMPLE 4 using prior art silver stearate as demonstrated by a reduced increase in Dₘₐₓ while maintaining Dₘᵢₙ-stability as measured both through a UV-filter and through a visible filter. The results in table 10 also show an increased stability of the photothermographic recording material of INVENTION EXAMPLE 4 to room lighting compared with the photothermographic recording material of COMPARATIVE EXAMPLE 4 using prior art silver stearate, as shown by a reduced increase in density measured through a UV-filter. The optical density measured through a UV-filter is relevant to the use of photothermographic materials for copying purposes in which UV-light is used. The photothermographic recording material of INVENTION EXAMPLE 4 only differs from that of COMPARATIVE EXAMPLE 4 in that it contains silver stearate with an increased crystallinity. This demonstrates the beneficial effect of increased silver stearate crystallinity on the stability of photothermographic recording materials.

**Table 10**

| Comparative example number | Silver stearate | | | fresh print characteristics upon printing for 10s at 105°C | | light box ΔDₘᵢₙ (UV) after 3d at 30°C/85%RH | shelf-life in dark ΔDₘₐₓ/ΔDₘᵢₙ (UV) after 3d at 35°C/80% RH |
|---|---|---|---|---|---|---|---|
| | type | coating weight [g/m²) | crystallinity | Dₘₐₓ (UV) | Dₘᵢₙ (UV) | unprocessed sheets | for printing at 105°C/10s |
| 4 | II | 5.55 | 1.76 | 4.95 | 0.25 | 0.55 | -0.70/-0.02 |

| Invention example nr | | | | | | | for printing at 110°C/10s |
|---|---|---|---|---|---|---|---|
| 4 | IV | 4.93 | 3.61 | 3.41 | 0.19 | 0.17 | -0.18/-0.17 |

**Table 11**

| Comparative example number | Silver stearate | | | fresh print characteristics upon printing for 10s at 105°C | | shelf-life in dark ΔDₘₐₓ/ΔDₘᵢₙ (vis) after 3d at 35°C/80%RH |
|---|---|---|---|---|---|---|
| | type | coating weight [g/m²] | crystallinity | Dₘₐₓ (VIS) | Dₘᵢₙ (VIS) | for printing at 105°C/10s |
| 4 | II | 5.55 | 1.76 | 1.45 | 0.08 | -0.23/-0.02 |

| Invention example nr | | | | | | for printing at 110°C/10s |
|---|---|---|---|---|---|---|
| 4 | IV | 4.93 | 3.61 | 2.13 | 0.12 | +0.06/-0.10s |

Having described in detail preferred embodiments of the current invention, it will now be apparent to those skilled in the art that numerous modifications can be made therein without departing from the scope of the invention as defined in the following claims.

## Claims

1. A recording material comprising a support and a thermosensitive element comprising silver stearate, an organic reducing agent therefor in thermal working relationship therewith and a binder, **characterized in that** said silver stearate is not associated with mercury and/or lead ions and when said recording material is irradiated with a copper Kα₁ X-ray source the ratio of the sum of the peak heights of the X-ray diffraction lines attributable to silver stearate at Bragg angles, 2Θ, of 3.62°, 5.45°, 7.30°, 9.04°, 10.97° and 12.71° to the sum of the peak heights of the X-ray diffraction lines at Bragg angles, 2Θ, of 25.60°, 35.16° and 43.40° of NIST standard 1976, rhombohedral Al₂O₃, determined with the same X-ray diffractometer in the same state of adjustment on a sample of said recording material and a sample of said NIST standard 1976 cut to fit a sample holder of said X-ray diffractometer, divided by the square root of the quantity of silver in said recording material, expressed in g per m², is greater than 2.2m/g^{0.5}.

2. Recording material according to claim 1, wherein said ratio divided by the square root of the quantity of silver in said recording material expressed in g per m² is greater than 3.0m/g^{0.5}.

3. Recording material according to claim 1 or 2, wherein said thermosensitive element is provided with a protective layer.

4. Recording material according to any of the preceding claims, wherein said thermosensitive element further comprises a photosensitive species capable upon exposure of forming a species capable of catalyzing reduction of said silver stearate.

5. A production process for a dispersion of particles of substantially light-insensitive organic silver salt containing silver stearate in a substantially solvent-free aqueous medium comprising the steps of: i) producing an aqueous dispersion of one or more organic acids containing stearic acid and an anionic surfactant; ii) substantially neutralizing said organic acids with aqueous alkali thereby forming organic acid salts containing a stearic acid salt; (iii) adding an aqueous solution of a silver salt to convert completely said organic acid salts into their silver salts containing silver stearate, **characterized in that** said anionic surfactant is present in a molar ratio with respect to organic acid greater than 0.15 and said silver salt is added to produce organic silver salt(s) at a rate between 0.025mol/mol organic silver salt(s) • min and 2.25mol/mol organic silver salt(s) • min.

6. Production process according to claim 5, wherein said anionic surfactant is an alkali or ammonium salt of an alkaryl sulfonic acid.

7. Production process according to claim 6, wherein said alkali or ammonium salt of an alkylarylsulfonic acid is an alkali or ammonium salt of an alkylbenzene sulfonic acid.

8. Production process according to any of claims 5 to 7 further comprising the step of subjecting said dispersion of particles of substantially light-insensitive organic silver salt(s) containing silver stearate to ultrafiltration.

9. Particles of substantially light-insensitive organic silver salt containing silver stearate producible according to any of claims 5 to 8.

10. A recording process comprising the steps of: (i) bringing an outermost layer of a recording material according to any of claims 1 to 3 into proximity with a heat source; and (ii) applying heat from said heat source imagewise to said recording material while maintaining proximity to said heat source to produce an image; and (iii) removing said recording material from said heat source.

11. Recording process according to claim 10, wherein said heat source is a thin film thermal head.

## Patentansprüche

1. Ein Aufzeichnungsmaterial mit einem Träger und einem wärmeempfindlichen Element, das Silberstearat, ein organisches Reduktionsmittel für das Silberstearat in thermischer wirksamer Beziehung dazu und ein Bindemittel enthält, **dadurch gekennzeichnet, daß** das Silberstearat nicht mit Quecksilber- und/oder Bleiionen kombiniert wird und bei Bestrahlung des Aufzeichnungsmaterials mit einer Kupfer-Kα₁-Röntgenstrahlungs-quelle das in g/m² ausgedrückte Verhältnis der Summe der Spitzenhöhen der Röntgenbeugungslinien von Silberstearat bei Braggschen Winkeln, 2Θ, von 3,62°, 5,45°, 7,30°, 9,04°, 10,97° und 12,71° zur Summe der Spitzenhöhen der Röntgenbeugungslinien bei Braggschen Winkeln, 2Θ, von 25,60°, 35,16° und 43,40°, die nach der NIST-Norm 1976 unter Verwendung von rhomboedrischem Al₂O₃ erhalten sind, wobei die Ermittlung mit demselben Röntgendiffraktometer mit gleichen Einstellungen auf einem Muster des Aufzeichnungsmaterials und einem Muster nach der NIST-Norm 1976 vorgenommen wird und die Muster so zugeschnitten sind, das sie in den Musterhalter des Röntgendiffraktometers passen, geteilt durch die Quadratwurzel der Menge Silber im Aufzeichnungsmaterial, mehr als 2,2 m/g^{0,5} beträgt.

2. Aufzeichnungsmaterial nach Anspruch 1, **dadurch gekennzeichnet, daß** das in g/m² ausgedrückte Verhältnis, geteilt durch die Quadratwurzel der Menge Silber im Aufzeichnungsmaterial, mehr als 3,0 m/g^{0,5} beträgt.

3. Aufzeichnungsmaterial nach Anspruch 1 oder 2, **dadurch gekennzeichnet, daß** das wärmeempfindliche Element mit einer Schutzschicht versehen ist.

4. Aufzeichnungsmaterial nach einem der vorstehenden Ansprüche, **dadurch gekennzeichnet, daß** das wärmeempfindliche Element weiterhin eine strahlungsempfindliche Substanz enthält, die bei Belichtung eine Substanz zu bilden vermag, die in der Lage ist, die Reduktion des Silberstearats zu katalysieren.

5. Ein durch die nachstehenden Schritte gekennzeichnetes Verfahren zur Herstellung einer Dispersion von Teilchen von wesentlich lichtunempfindlichem organischem Silberstearat enthaltendem Silbersalz in einem wesentlich lösungsmittelfreien wäßrigen Medium :
(i) Anfertigung einer wäßrigen Dispersion einer oder mehrerer organischer Säuren, einschließlich stearinsäure, und eines anionischen Tensids,
(ii) weitgehendes Neutralisieren der organischen Säuren mit wäßrigem Alkali, wobei organische Säuresalze, einschließlich eines stearinsäuresalzes, gebildet werden,
(iii) Zugabe einer wäßrigen Lösung eines Silbersalzes, um die organischen Säuresalze völlig in deren Silberstearat enthaltende Silbersalze umzuwandeln,
**dadurch gekennzeichnet, daß** das anionische Tensid in einem Molverhältnis, bezogen auf die organische Säure, von mehr als 0,15 enthalten ist und das Silbersalz bei einer Rate zwischen 0,025 Mol/Mol organische(s) Silbersalz(e) · Min. und 2,25 Mol/Mol organische(s) Silbersalz(e) · Min. zugegeben wird, um ein oder mehrere organische Silbersalze zu bilden.

6. Herstellungsverfahren nach Anspruch 5, **dadurch gekennzeichnet, daß** das anionische Tensid ein Alkalioder Ammoniumsalz einer Alkarylsulfonsäure ist.

7. Herstellungsverfahren nach Anspruch 6, **dadurch gekennzeichnet, daß** das Alkali- oder Ammoniumsalz einer Alkylarylsulfonsäure ein Alkali- oder Ammoniumsalz einer Alkylbenzolsulfonsäure ist.

8. Herstellungsverfahren nach einem der Ansprüche 5 bis 7, das einen weiteren Schritt umfaßt, in dem die Dispersion der Teilchen des (der) wesentlich lichtunempfindlichen organischen silberstearathaltigen Silbersalze(s) ultrafiltriert wird.

9. Teilchen eines wesentlich lichtunempfindlichen organischen silberstearathaltigen Silbersalzes, die nach einem der Ansprüche 5 bis 8 anfertigbar sind.

10. Ein durch die nachstehenden Schritte gekennzeichnetes Aufzeichnungsverfahren :
(i) Anordnen einer Außenschicht eines nach einem der Ansprüche 1 bis 3 definierten Aufzeichnungsmaterials in der Nähe einer Heizquelle,
(ii) bildmäßige Beaufschlagung des Aufzeichnungsmaterials mit von der Heizquelle gelieferter Wärme zur Herstellung eines Bildes, wobei das Aufzeichnungsmaterial in der Nähe der Heizquelle gehalten wird, und
(iii) Entfernen des Aufzeichnungsmaterials von der Heizquelle.

11. Aufzeichnungsverfahren nach Anspruch 10, **dadurch gekennzeichnet, daß** die Heizquelle ein Dünnfilmthermokopf ist.

## Revendications

1. Matériau d'enregistrement comprenant un support et un élément thermosensible comprenant du stéarate d'argent, un agent de réduction organique qui lui est destiné et disposé en liaison de travail thermique avec lui, et un liant, **caractérisé en ce que** ledit stéarate d'argent n'est pas associé à des ions de mercure et/ou de plomb et **en ce que** ledit matériau d'enregistrement est exposé à un rayonnement avec une source à rayons X à cible en cuivre à Kα₁, le rapport de la somme des hauteurs de pics des lignes de diffraction des rayons X qui peuvent être attribuées au stéarate d'argent à des angles de Bragg, 2Θ de 3,62°, de 5,45°, de 7,30°, de 9,04°, de 10,97° et de 12,71° à la somme des hauteurs de pics des lignes de diffraction des rayons X à des angles de Bragg, 2Θ de 25,60°, de 35,16° et de 43,40° du Al₂O₃ rhomboédrique de la norme NIST 1976, déterminé avec le même diffractomètre des rayons X dans le même état de réglage sur un échantillon dudit matériau d'enregistrement et sur un échantillon de ladite norme NIST 1976 découpés pour venir s'insérer dans le support d'échantillon dudit diffractomètre des rayons X, divisé par la racine carrée de la quantité d'argent dans le matériau d'enregistrement exprimée en g par m², est supérieur à 2,2 m/g^{0,5}.

2. Matériau d'enregistrement selon la revendication 1, dans lequel ledit rapport, divisé par la racine carrée de la quantité d'argent dans le matériau d'enregistrement exprimée en g par m², est supérieur à 3,0 m/g^{0,5}.

3. Matériau d'enregistrement selon la revendication 1 ou 2, dans lequel ledit élément thermosensible est muni d'une couche de protection.

4. Matériau d'enregistrement selon l'une quelconque des revendications précédentes, dans lequel ledit élément thermosensible comprend en outre une espèce photosensible capable, lors de l'exposition, de former une espèce capable de catalyser la réduction dudit stéarate d'argent.

5. Procédé de préparation pour une dispersion de particules d'un sel d'argent organique essentiellement non photosensible contenant du stéarate d'argent dans un milieu aqueux essentiellement exempt de solvant, comprenant les étapes consistant à : (i) préparer une dispersion aqueuse d'un ou de plusieurs acides organiques, y compris de l'acide stéarique, et d'un agent tensioactif anionique ; (ii) neutraliser de manière substantielle que lesdits acides organiques avec un alcali aqueux pour ainsi former des sels d'acides organiques y compris un sel de l'acide stéarique ; (iii) ajouter une solution aqueuse d'un sel d'argent pour transformer complètement lesdits sels d'acides organiques en leurs sels d'argent, y compris le stéarate d'argent, **caractérisé en ce que** ledit agent tensioactif anionique est présent dans un rapport molaire par rapport à l'acide organique, supérieur à 0,15 et ledit sel d'argent est ajouté pour obtenir un sel ou des sels d'argent organique à un débit entre 0,025 mole/mole de sel(s) d'argent organique(s) par minute et de 2,25 moles/mole de sels) d'argent organique(s) par minute.

6. Procédé de préparation selon la revendication 5, dans lequel ledit agent tensioactif anionique est un sel de métal alcalin ou d'ammonium d'un acide alkarylsulfonique.

7. Procédé de préparation selon la revendication 6, dans lequel ledit sel de métal alcalin d'ammonium d'un acide alkarylsulfonique est un sel de métal alcalin ou d'ammonium d'un acide alkylbenzène-sulfonique.

8. Procédé de préparation selon l'une quelconque des revendications 5 à 7, comprenant en outre l'étape consistant à soumettre à une ultrafiltration ladite dispersion de particules de sel(s) d'argent organique(s) essentiellement non photosensible(s) contenant du stéarate d'argent.

9. Particules d'un sel d'argent organique essentiellement non photosensible contenant du stéarate d'argent que l'on peut obtenir conformément à l'une quelconque des revendications 5 à 8.

10. Procédé d'enregistrement comprenant les étapes consistant à : (i) amener une couche d'un matériau d'enregistrement située le plus à l'extérieur selon l'une quelconque des revendications 1 à 3, à proximité d'une source de chaleur ; et (ii) appliquer de la chaleur à partir de ladite source de chaleur en forme d'image sur ledit matériau d'enregistrement tout en maintenant ce dernier à proximité de ladite source de chaleur pour produire une image ; et (iii) retirer ledit matériau d'enregistrement de ladite source de chaleur.

11. Procédé d'enregistrement selon la revendication 10, dans lequel ladite source de chaleur est une tête thermique en film mince.
